# EUROPEAN PATENT APPLICATION

(11) **EP 4 803 524 A1**
(43) Date of publication of application: **09.09.2026**
(21) Application number: 25220971.3
(22) Date of filing: 05.12.2025
(51) Int. Cl.: C07D 493/04, C08K 5/00, C08L 23/00

(54) **POLYMORPHIC FORM B OF 3-(3,5-DI-TERT-BUTYL-4-HYDROXY-PHENYL)-PROPIONIC ACID 6-[3-(3,5-DI-TERT-BUTYL-4-HYDROXY-PHENYL)-PROPIONYLOXY]-HEXAHYDRO-FURO[3,2-B]FURAN-3-YL ESTER AND PROCESS FOR PREPARATION THEREOF**

(30) Priority: 04.03.2025 IN 202541019389
(71) Applicant: Hindustan Petroleum Corporation Limited, Bengaluru 560067 (IN)
(72) Inventor: HALDAR, Ujjal, 560067 Bengaluru (IN); RAO, Pedada Srinivasa, 560067 Bengaluru (IN); SAMARTH, Nikesh, 560067 Bengaluru (IN); THIRUPATHY, Dharun, 560067 Bengaluru (IN); KUMAR, Vinoth, 560067 Bengaluru (IN); PATIL, Yogesh Popatrao, 560067 Bengaluru (IN); CHELLIAHN, Bennet, 560067 Bengaluru (IN); SHESHACHALA, Srinivasa Narasimha, 560067 Bengaluru (IN)
(74) Representative: Wallinger Ricker Schlotter PartGmbB

(57) **Abstract**

The present disclosure relates to a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73. The present disclosure also relates to a process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester. The polymorphic Form B is used for thermos-oxidative stabilization of polyolefins. The present disclosure also provides a composition for thermo-oxidative stabilization of polyolefins. All the properites such as oxygen induction time (OIT), change in melt flow index (MFR), yellowness index (YI), Change in yellowness index have been improved without compromising the mechanical property.

## Description

### FIELD OF THE INVENTION

The present disclosure generally relate to a field of thermo-oxidative stabilization of polyolefins. More particularly, the present disclosure relates to a polymorphic Form-B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester. Further, the present disclosure also relates to a process of preparation of a polymorphic Form-B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester.

### BACKGROUND

Background description includes information that may be useful in understanding the present invention. It is not an admission that any of the information provided herein is prior art or relevant to the presently claimed invention, or that any publication specifically or implicitly referenced is prior art.

Polyolefins normally undergo high temperature manufacturing and fabrication operations and are susceptible to oxidation during various stages of their lifecycle. Antioxidants are used to inhibit the oxidative damage that is ultimately responsible for loss of physical properties, embrittlement and premature failure. The effectiveness of thermal stabilizers or antioxidants in stabilizing polymers depends on many factors including solubility, dispersion, ability to stabilize different polymer matrices, evaporation or volatilization during processing, conditions of use, and recycling.

Conventionally, various types of primary antioxidants, mostly hindered phenol based antioxidants, have been used to serve this purpose. For example, commercial antioxidant Pentaerythritol tetrakis(3,5-di-tert-butyl-4-hydroxyhydrocinnamate) is based on the family of hindered phenols like 4-substituted-2,6-ditertiary butyl phenols. Notably, most of the conventional primary antioxidants are derived from synthetic organic molecules. However, stabilization of polyolefins with synthetic antioxidants can be used only upto a certain concentration, beyond which, if used, they make the polyolefins unfit for food contact applications. Typical hindered phenols tend to migrate to the surfaces of polymeric articles. Plastics that are used for food packaging have come under scrutiny because synthetic antioxidants may contaminate food and serve as a source of unknown cytotoxicity. Few details are known about the toxicity and migration of the transformation products of such antioxidants produced during processing and application. Other environmental concerns arise from the presence of trace levels of heavy metal catalyst used for antioxidant synthesis.

The aforesaid shortcomings as well as economic and environmental factors have led to growing interest in using greener, sustainable materials in place of synthetic counterparts as feedstocks. Such sustainable molecules provide the benefits of biodegradability, reduce wear to processing equipment, and low cost. In light of these benefits as well as concerns over the use of synthetic antioxidants, numerous bio-based phenols such as caffeic acid, ascorbic acid (vitamin C), C-tocopherol (vitamin E), curcumin, quercetin, and B-carotenes have reportedly been incorporated into different packaging materials as thermal stabilizers. For example, Al-Malaika et al. (Polymer Degradation and Stability 73 (2001) 491-503) examined the efficacy of the commercially-available compound vitamin E as a melt stabilizer for low density polyethylene (LDPE); Tatraalai et al. (European Polymer Journal 49 (2013) 1196-1203) similarly investigated the effect of curcumin, found in turmeric and other roots, on the processing stability of PE, wherein using various characterization techniques, they demonstrated that PE with 0.1 wt % curcumin showed superior thermo-oxidative stability compared to that for PE with 0.1 wt % Pentaerythritol tetrakis(3,5-di-tert-butyl-4-hydroxyhydrocinnamate); Cerruti et al. (US Pub. No.: US20170058108A1) used ball milling and solvent extraction to obtain antioxidant fractions from grape seeds and tomato skins, incorporation of such carotenoid antioxidant fractions were reported to have positive effects on PP thermal stability; Maraveas et al. (Polymers 2021, 13(15), 2465; available at https://doi.org/10.3390/polym13152465) briefly described various naturally occurring small molecule or polymers such as lignin, and graft polymers, dopamine, and polydopamine, inulin, quercetin, limonene, and vitamins and their potential application for stabilization of polymers.

IN202241062060 discloses Form-A of 3-(3,5-di-tert-butyl-4-hydroxyphenyl)-propionic acid 6-[3-(3,5-di-tert-butyl-4-hydroxy-phenyl)-propionyloxy]-hexahydro-furo[3,2-b]furan-3-yl ester as a primary antioxidant. The Form-A has a melting point of 55 to 60 °C and having low crystallinity in nature. Form-A is highly hygroscopic and exposing of air and moisture forms cake/lump. The compound 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester has two chiral centers at the fused furan system (C-3 and C-6). The stereochemistry at these positions is often defined as (3R,6R) or (3S,6S) or their enantiomers (3R,6S) / (3S,6R). Without additional information (such as NMR, X-ray, or synthesis details), the specific configuration (R or S) at these centers remains ambiguous. Thus, there is a possibility to develop a new polymorphic Form which can overcome the drawbacks of the Form-A.

Despite rigorous research done in the technical field of thermo-oxidative stabilization of polyolefins, the conventional anti-oxidants, specifically, the primary anti-oxidants, suffer from one or more shortcomings. Consequently, there is a persistent need in the state of the art for bio-based primary antioxidants for thermal oxidative stabilization of polyolefins. Need is also felt of a process for preparation of bio-based primary antioxidants.

Each of the documents referred in the background section are incorporated herein, in its entirety, by way of reference. Further, none of the abovementioned documents are to be construed as relevant prior-art for the invention as embodied in the present disclosure. The sole intention of referring to and providing the abovementioned documents is to highlight some of the work already done in the technical field of thermo-oxidative stabilization of polyolefins.

### OBJECTS OF THE INVENTION

An object of the present disclosure is to provide a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester.

Another object of the present disclosure is to provide a process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester.

Further object of the present disclosure is to provide a novel polymorph having highly hydrophobic and low moisture capability even after long preservation.

Still further object of the present disclosure is to provide use of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester for thermo-oxidative stabilization of polyolefins.

Yet another object of the present disclosure is to provide composition for thermo-oxidative stabilization of polyolefins.

### SUMMARY OF THE INVENTION

This summary is provided to introduce a selection of concepts in a simplified form that are further described below in Detailed Description section. This summary is not intended to identify key features or essential features of the claimed subject matter, nor is it intended to be used as an aid in determining the scope of the claimed subject matter.

An aspect of the present disclosure is to provide a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

Another aspect of the present disclosure is to provide a process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, the process comprising: a) heating an isosorbide and a methyl 3-(3,5-di-tert-butyl-4-hydroxyphenyl)propionate in presence of a catalyst and a first solvent to obtain a crude product; b) adding a second solvent to the crude product to obtain a solution; c) introducing an adsorbent to the solution with stirring to obtain a mixture; d) filtering the mixture to obtain a filtrate; e) adding a seeding agent in the filtrate with stirring to obtain a solid product; and f) filtering the solid product followed by drying to obtain a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

Further aspect of the present disclosure is to provide use of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester for thermo-oxidative stabilization of polyolefins, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

Still further aspect of the present disclosure is to provide a composition for thermo-oxidative stabilization of polyolefins, said composition comprising: (a) a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73; (b) a secondary anti-oxidant; and (c) an acid scavenger.

Various objects, features, aspects and advantages of the inventive subject matter will become more apparent from the following detailed description of preferred embodiments, along with the accompanying drawing figures in which like numerals represent like components.

### BRIEF DESCRIPTION OF THE DRAWINGS

The accompanying drawings are included to provide a further understanding of the present disclosure and are incorporated in and constitute a part of this specification. The drawings illustrate exemplary embodiments of the present disclosure and together with the description, serve to explain the principles of the present disclosure.
Figure 1 illustrates PXRD profile of Form-A (old form), and Form-B (new form).
Figure 2 illustrates Form-A (Crystalline: 7%), and Form-B (crystalline: 89%).
Figure 3 illustrates DSC profile of Form-A (old form), and Form-B (new form).
Figure 4 illustrates (A) TGA profile of Form-A (old form) and (B) TGA profile of Form-B (new form).

### DETAILED DESCRIPTION OF THE INVENTION

The following is a detailed description of embodiments of the disclosure depicted in the accompanying drawings. The embodiments are in such detail as to clearly communicate the disclosure. However, the amount of detail offered is not intended to limit the anticipated variations of embodiments; on the contrary, the intention is to cover all modifications, equivalents, and alternatives falling within the spirit and scope of the present disclosure as defined by the appended claims.

Each of the appended claims defines a separate invention, which for infringement purposes is recognized as including equivalents to the various elements or limitations specified in the claims. Depending on the context, all references below to the "invention" may in some cases refer to certain specific embodiments only. In other cases, it will be recognized that references to the "invention" will refer to subject matter recited in one or more, but not necessarily all, of the claims.

Unless the context requires otherwise, throughout the specification which follow, the word "comprise" and variations thereof, such as, "comprises" and "comprising" are to be construed in an open, inclusive sense that is as "including, but not limited to."

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

As used in this specification and the appended claims, the singular forms "a," "an," and "the" include plural referents unless the content clearly dictates otherwise. It should also be noted that the term "or" is generally employed in its sense including "and/or" unless the content clearly dictates otherwise.

In some embodiments, the numbers expressing quantities of ingredients, properties such as concentration, reaction conditions, and so forth, used to describe and claim certain embodiments of the invention are to be understood as being modified in some instances by the term "about." Accordingly, in some embodiments, the numerical parameters set forth in the written description are approximations that can vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the invention are approximations, the numerical values set forth in the specific examples are reported as precisely as practicable. The recitation of ranges of values herein is merely intended to serve as a shorthand method of referring individually to each separate value falling within the range.

Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided with respect to certain embodiments herein is intended merely to better illuminate the invention and does not pose a limitation on the scope of the invention otherwise claimed. No language in the specification should be construed as indicating any non-claimed element essential to the practice of the invention.

The headings and abstract of the invention provided herein are for convenience only and do not interpret the scope or meaning of the embodiments.

Reference will now be made in detail to the exemplary embodiments of the present invention, examples of which are illustrated in the accompanying drawings.

The following description provides different examples and embodiments of the inventive subject matter. Although each embodiment represents a single combination of inventive elements, the inventive subject matter is considered to include all possible combinations of the disclosed elements. Thus, if one embodiment comprises elements A, B, and C, and a second embodiment comprises elements B and D, then the inventive subject matter is also considered to include other remaining combinations of A, B, C, or D, even if not explicitly disclosed.

All percentages, ratios, and proportions used herein are based on a weight basis unless otherwise specified.

Various terms as used herein are shown below. To the extent a term used in a claim is not defined below, it should be given the broadest definition persons in the pertinent art have given that term as reflected in printed publications and issued patents at the time of filing.

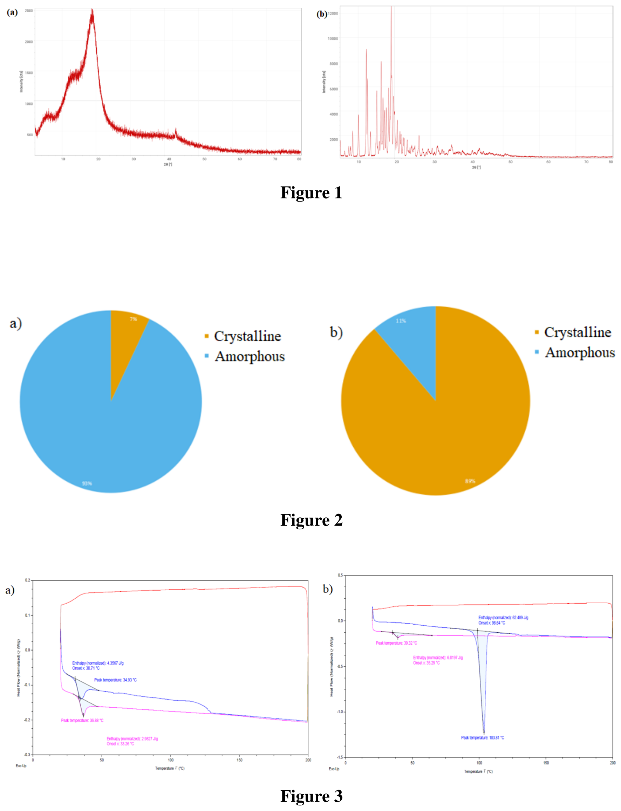

An embodiment of the present disclosure provides a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73. The characteristic peaks are as shown in Figure 1.

In an embodiment, the polymorphic Form B has a melting point ranging from 103 to 106 °C.

In an embodiment, the polymorphic Form B has a Themo gravimetric analysis (TGA) T_{d,5%} about 292 °C.

In some embodiment, the polymorphic Form B has more than 85 % crystallinity. In an embodiment, the polymorphic Form-B has 89% crystallinity and 11 % amorphous. Figure 2 shows that the Form-A has a crystalline of 7% whereas Form-B has a crystalline of 89%.

In some embodiment, the polymorphic Form B is highly hydrophobic and having low moisture capability even after 12 to 18 months preservation.

Another embodiment of the present disclosure provides a process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, the process comprising: a) heating an isosorbide and a methyl 3-(3,5-di-tert-butyl-4-hydroxyphenyl)propionate in presence of a catalyst and a first solvent to obtain a crude product; b) adding a second solvent to the crude product to obtain a solution; c) introducing an adsorbent to the solution with stirring to obtain a mixture; d) filtering the mixture to obtain a filtrate; e) adding a seeding agent in the filtrate with stirring to obtain a solid product; and f) filtering the solid product followed by drying to obtain a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

In some embodiment, the isosorbide has an amount ranging from 0.5 to 1.5 mol %. Preferably, the amount is 1 mol%.

In some embodiment, the methyl 3-(3,5-di-tert-butyl-4-hydroxyphenyl)propionate has an amount ranging from 1.5 to 2.5 mol %. Preferably, the amount is 1.95 mol%.

In some embodiment, the catalyst is selected from: (i) a tin based catalyst selected from the group consisting of stannous chloride (SC), dibutyl tin diacetate (DBTDA), dibutyl tin oxide (DBTO), dibutyl tin dialutarte (DBTDL), tin-2-ethylhexanoate (THE), butyl stannoic acid (BTA), butyltin hydroxide-oxide (BTA), and mixtures thereof; (ii) a salt of non-volatile inorganic acid selected from the group consisting of NaH₂PO₃, NaH₂PO₄, KH₂PO₄, CsH₂PO₄, Zn(CH₃CO₂)₂, and mixtures thereof; and (iii) sodium hydroxide, sodium carbonate, lithium hydroxide, lithium carbonate, tetraalkylammonium hydroxide, tetraalkyl ammonium carbonate, titanium alkoxide, lead alkoxide, metal acetate, 4-dimethyl amino pyridine, titanium alkoxide, synthetic hydrolacite, mixed metal oxide and mixtures thereof and has an amount ranging from 0.01 to 0.1 mol %. Preferably, the amount is 0.05 mol%.

In some embodiment, the step a) is carried out at a temperature ranging from 110 to 140 °C for a time period ranging from 10 to 14 hours and at a pressure ranging from 10 to 100 mbar.

In some embodiment, the second solvent is selected from a group consisting of pentane, hexane, heptane, cyclo hexane, xylene, toluene and combination thereof and has an amount ranging from 35 to 45 mol %. Preferably, the amount is 40 mol%.

In some embodiment, the second solvent is added to the crude product at a temperature ranging from 60 to 70 °C. Preferably, the temperature is 65 °C.

In some embodiment, the adsorbent is selected from a group consisting of INF-04, silica gel, activated alumina, zeolites, synthetic polymeric resins and combination thereof and has an amount ranging from 2 to 3 mol %. Preferably, the amount is 2.5 mol%.

In some embodiment, the step c) is carried out at a temperature ranging from 25 to 35 °C with stirring at a speed ranging from 50 to 200 RPM for a time period ranging from 30 to 90 minutes.

In some embodiment, the adsorbent treatment of step c) is repeated until the acid impurity is reduced to less than 0.50 %.

In some embodiment, the seeding agent is pure 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and has an amount ranging from 0.001 to 0.005 mol%.

In some embodiment, the stirring in step e) is carried out at a temperature ranging from 25 to 35 °C at a speed ranging from 50 to 200 RPM for a time period ranging from 6 to 24 hours.

Still another embodiment of the present disclosure provides use of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester for thermo-oxidative stabilization of polyolefins, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

Yet another embodiment of the present disclosure provides composition for thermo-oxidative stabilization of polyolefins, said composition comprising: (a) a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73; (b) a secondary anti-oxidant; and (c) an acid scavenger.

In some embodiment, the composition comprises the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the secondary anti-oxidant in a weight ratio ranging from 10:1 to 1:10. In a preferred embodiments, the weight ratio ranging from 8:1 to 1:10 or 5:1 to 1:8 or 5:1 to 1:5 or 3:1 to 1:4.

In some embodiment, the weight ratio between the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the acid scavenger ranges between 5.0:1.0 to 1.0:5.0. In a preferred embodiments, the weight ratio ranges between 4.0:1.0 to 1.0:4.0 or 3.0:1.0 to 1.0:4.0 or 3.0:1.0 to 1.0:3.0 or 2.0:1.0 to 1.0:3.0.

In some embodiment, the secondary anti-oxidant is selected from phosphanite based secondary antioxidant and phosphite based secondary antioxidant.

In some embodiment, the acid scavenger is selected from hydrotalcite type acid scavengers, metal stearates type acid scavengers and mixtures thereof.

While the foregoing describes various embodiments of the disclosure, other and further embodiments of the disclosure may be devised without departing from the basic scope thereof. The invention is not limited to the described embodiments, versions or examples, which are included to enable a person having ordinary skill in the art to make and use the invention when combined with information and knowledge available to the person skilled in the art.

### EXAMPLES

The present disclosure is further explained in the form of the following examples. However, it is to be understood that the examples are merely illustrative and are not to be taken as limitations upon the scope of the invention. Various changes and modifications to the disclosed embodiments will be apparent to those skilled in the art. Such changes and modifications may be made without departing from the scope and spirit of the present invention.

### Example 1:

### (A) Method of preparation of polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester

The reaction was carried out in a 1 L double-jacketed glass reactor equipped with an overhead stirrer, condenser, and a Dean-Stark apparatus. Prior to the reaction, the reactor was thoroughly cleaned and dried under a nitrogen atmosphere. Dianhydro-D-glucitol (Isosorbide) (1 mol%), methyl 3-(3,5-di-tert-butyl-4-hydroxyphenyl)propionate (1.95 mol%), and dibutyltin oxide (0.05 mol%) were introduced into the reactor under nitrogen atmosphere. Toluene (20 mol%) was used as the reaction solvent. The reaction mixture was refluxed for 12 hours, during which methanol as a by-product, was continuously collected in the Dean-Stark apparatus. The reaction progress was monitored by High-Performance Liquid Chromatography (HPLC). Upon achieving 60-70% product conversion, the solvent was removed under vacuum. The reaction temperature was then gradually increased to 140 °C, while simultaneously applying a vacuum of approximately 10-15 mbar. The reaction was allowed to proceed under these conditions for an additional 16 hours. At the end of the reaction, a brown viscous crude product was obtained with a conversion rate of 90-91%. After the completion of the reaction (crude product), the temperature was gradually decreased to 60-70°C, followed by the addition of hexane (40 mol%). The reaction mixture was stirred until a clear solution was obtained. The solution was then cooled to 25-35 °C and adsorbent (INF-04) (2.5 mol%) was introduced. The mixture was stirred at 25-35 °C for 60 minutes, after which the reaction mass was filtered. This adsorbent treatment was repeated until the acid impurity was reduced to less than 0.50 %. The adsorbent was helped to removal of impurities and improved the colour of the solution. The filtrate was transferred into the reactor, and pure product was added as a seeding material. After 6-8 hours of stirring at room temperature, white precipitation was observed. The stirring was continued for an additional 16 hours to maximize product yield. The resulting solid was filtered and dried under vacuum, yielding the new polymorph as an off-white product.

### (B) Characterization

The obtained product was characterized using spectroscopic techniques, including NMR, FTIR, Mass Spectrometry, XRD, DSC and TGA.

### (i) XRD

The polymorph Form B was characterize by X-ray powder diffraction pattern (CuKα) having peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73 as shown in Figure 1.

### (ii) DSC

The melting point of the new polymorph was determined by DSC (m.p. = 103-106 °C) as shown in Figure 3.

### (C) Stability of Form-B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester

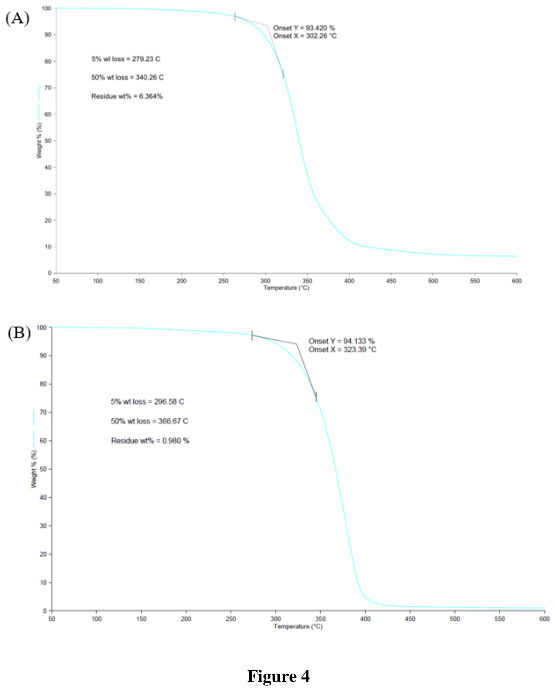

The stability of the polymorphic Form-B prepared above was investigated by thermogravimetric (TGA) analysis from a range of 20-600 °C at a rate of 10 °C/min. Figure 4 shows an exemplary TGA curve of the polymorphic Form-A and Form-B. Figure 4 A shows that the polymorphic Form A has a themo gravimetric analysis (TGA) T_{d,5%} about 279 °C and T_{d,50%} about 340 °C respectively under N₂ atmosphere. Whereas the polymorphic Form B has a themo gravimetric analysis (TGA) T_{d,5%} about 297 °C and T_{d,50%} about 367 °C respectively under N₂ atmosphere (Figure 4B), making it evident that the antioxidant compound is able to sustain higher temperatures.

### Example 2: Composition for stabilization of polyolefins

Form-A and Form-B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester [code: "HP-AO (Form-A)" and "HP-AO (Form-B)"] respectively as a primary anti-oxidant. Reference/standard anti-oxidant - Irganox 1010 (code: "Reference"), a secondary antioxidant - Irgafos 168, and an acid scavenger - DHT-4A^{®} were added to polypropylene to prepare PP compositions. PP compositions were prepared by mixing the polypropylene powder along with the HP-AO (Form-A) and HP-AO (Form-B) as a primary antioxidant, phosphite/phosphonite as a secondary antioxidant, and acid scavenger in the requisite amounts in a high-speed mixer for 10-15 minutes. Composition details are provided in Table 1 below:

**Table 1: Compositions for thermo-oxidative stabilization of polyolefins.**

| Additives | Reference | HP-AO (Form-A) | HP-AO (Form-B) |
|---|---|---|---|
| Irganox 1010 (ppm) | 450 | - | |
| HP-AO (Form-A) (ppm) | - | 450 | |
| HP-AO (Form-B) (ppm) | - | - | 450 |
| Irgafos 168 (ppm) | 900 | 900 | 900 |
| DHT4A (ppm) | 300 | 300 | 300 |

| | | | |
|---|---|---|---|
| • Non-stabilized PP: MFI 6.1g/10min used for evaluation | | | |

### (A) Studies to assess thermo-oxidative stabilization of polypropylene

The performance of the polypropylene compositions ("HP-AO (Form-A)", "HP-AO (Form-B)" and "Reference") prepared in Example 2 above was evaluated using twin-screw extruder. Temperature profile maintained in the extruder (Omega 25, Screw dia.: 25 mm, L/D: 44) was from 150 to 250 °C and screw rotation was 50-150 rpm. Table 2 below provides temperature profile of Twin-Screw extruder during extrusion.

**Table 2: Temperature profile of Twin-Screw extruder during extrusion**

| | | | |
|---|---|---|---|
| Barrel Temperature | 150-250 °C | Out put | 15 kg/hr |
| Die Temperature | 200-210 °C | RPM | 300 |
| Melt Temperature | 212-250 °C | - | - |

Five repetitive extrusions were done and coded as I pass, II pass, III pass, IV pass and V pass. After each extrusion, all the properties were measured such as melt flow index (MFI), oxygen induction time (OIT), yellowness index (YI), and thermal ageing. Thermal ageing was performed at 150 °C in air circulated oven from 0 to 72 hours. After thermal aging, the mechanical properties and yellowness index were measured at each 24 hour time intervals.

In order to understand the melt flow rate (MFR) of PP compositions viz. Reference, HP-AO (Form-A) and HP-AO (Form-B), MFR for all five repetitive extrusions were determined keeping other parameters identical, results wherefor are provided in Table 3 below.

It is clear from Table 3 that HP-AO (Form-B) is better control on MFR, as Change of MFR is minimum in case of HP-AO (Form-B).

**Table 3: Change in MFR with multiple extrusion.**

| Sample | Change in MFR (g/10 min) | | | | | MFR Difference between V & I Pass |
|---|---|---|---|---|---|---|
| | I Pass | II Pass | III Pass | IV Pass | V Pass | |
| Reference | 4.5 | 5.4 | 8.2 | 9.1 | 11.4 | 6.9 |
| HP-AO (Form-A) | 4.5 | 5.4 | 6.8 | 8.7 | 11.4 | 6.9 |
| HP-AO (Form-B) | 4.5 | 5.3 | 6.5 | 8.1 | 10.3 | 6.0 |

Oxidative Induction Time (OIT) values of PP composites after each extrusion and its difference of I and V pass for both Refrence, HP-AO (Form-A) and HP-AO (Form-B) were determined, results wherefor are provided in Table 4 below. From Table 4, it is clear that HP-AO (Form-B has higher OIT value compared to benchmark as well as Form-A (i.e.; old form).

**Table 4: Evaluation of oxygen induction time (OIT).**

| Sample | Oxidative Induction Time (min) | | | | |
|---|---|---|---|---|---|
| | I Pass | II Pass | III Pass | IV Pass | V Pass |
| Reference | 4.56 | 4.12 | 3.97 | 3.54 | 3.45 |
| HP-AO (Form-A) | 4.55 | 4.66 | 4.34 | 3.75 | 3.54 |
| HP-AO (Form-B) | 5.87 | 4.73 | 3.7 | 3.35 | 3.81 |

Yellowness index (YI) values of PP composites after each extrusion and its difference of I and V pass for both Refrence, HP-AO (Form-A) and HP-AO (Form-B) were determined using spectrophotometer, results wherefor are provided in Table 5 below. From Table 5, It is clear that HP-AO (Form-B) has slightly higher YI difference compared to the reference as well as old form.

**Table 5: Change in yellowness index (YI).**

| Sample | Change in Yellowness Index with multi-pass | | | YI Difference between V & I Pass |
|---|---|---|---|---|
| | I Pass | III Pass | V Pass | |
| Reference | -11.12 | -4.49 | -1.49 | 12.6 |
| HP-AO (Form-A) | -11.28 | -6.64 | -4.54 | 15.8 |
| HP-AO (Form-B) | -11.13 | -5.61 | -2.88 | 14.0 |

To check whether the YI changes after heat ageing treatment for different time intervals, Refrence, HP-AO (Form-A) and HP-AO (Form-B) were heated at 150 °C for 24, 48, and 72 hours in air circulated oven. From Table 6, it is clear that HP-AO (Form-B) has lower YI difference compared to the reference as well as old form. Therefor, this new form (Form-B) has better control while ageing.

**Table 6: Change in YI after thermal ageing study**

| Sample | Change in Yellowness Index After Thermal Ageing | | | Change in YI (Before ageing - After 72 hrs) |
|---|---|---|---|---|
| | I Pass | III Pass | V Pass | |
| Reference | -24.97 | -24.14 | -22.7 | 13.68 |
| HP-AO (Form-A) | -24.96 | -24.48 | -23.78 | 12.5 |
| HP-AO (Form-B) | -25.67 | -24.66 | -22.84 | 11.71 |

Heat aged specimen of Refrence, HP-AO (Form-A) and HP-AO (Form-B) were investigated for mechanical properties, results wherefor are provided in Table 7 below. It can be seen in Table 7 that there is no change in mechanical property.

**Table 7: Change in mechanical property.**

| Properties | Unit | Reference | | HP-AO (Form-A) | | HP-AO (Form-B) | |
|---|---|---|---|---|---|---|---|
| | | 0 hr | 72 hr | 0 hr | 72 hr | 0 hr | 72 hr |
| Tensile Strength at Yield | MPa | 34.7 | 33.3 | 34.5 | 33.3 | 34.9 | 33.2 |
| Elongation at Yield | % | 15.9 | 11.4 | 16.9 | 11 | 16.8 | 11.1 |
| Tensile Strength at Break | MPa | 14.9 | 31.1 | 15.8 | 30.6 | 18.5 | 30.1 |
| Elongation at Break | % | 104 | 203 | 125 | 201 | 136 | 167 |

The foregoing examples are merely illustrative and are not to be taken as limitations upon the scope of the invention. Various changes and modifications to the disclosed embodiments will be apparent to those skilled in the art. Such changes and modifications may be made without departing from the scope of the invention.

### ADVANTAGES OF THE PRESENT INVENTION

Polymorph Form-B has better control on MFR, as change of MFR is minimum in case of polymorph Form-B.

Polymorph Form-B has higher OIT value compared to benchmark as well as polymorph Form-A.

Polymorph Form-B has slightly higher YI difference compared to the reference as well as polymorph Form-A.

Polymorph Form-B has lower YI difference (Before ageing - After 72 hrs) compared to the reference as well as Polymorph Form-A. Therefore, this new form (Form-B) has better control while ageing.

Polymorph Form-B shows no change in mechanical property indicating no compromisation with the mechanical property.
We herewith provide, in particular, the following item list:
1. A polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.
2. The polymorphic Form B as claimed in item 1, wherein the polymorphic Form B has a melting point ranging from 103 to 106 °C.
3. The polymorphic Form B as claimed in item 1, wherein the polymorphic Form B has a Themo gravimetric analysis (TGA) T_{d,5%} about 297 °C.
4. The polymorphic Form B as claimed in item 1, wherein the polymorphic Form B has more than 85 % crystallinity.
5. The polymorphic Form B as claimed in item 1, wherein the polymorphic Form B is highly hydrophobic and having low moisture capability even after 12 to 18 months preservation.
6. A process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, the process comprising:
   a) heating an isosorbide and a methyl 3-(3,5-di-tert-butyl-4-hydroxyphenyl)propionate in presence of a catalyst and a first solvent to obtain a crude product;
   b) adding a second solvent to the crude product to obtain a solution;
   c) introducing an adsorbent to the solution with stirring to obtain a mixture;
   d) filtering the mixture to obtain a filtrate;
   e) adding a seeding agent in the filtrate with stirring to obtain a solid product; and
   f) filtering the solid product followed by drying to obtain a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.
7. The process as claimed in item 6, wherein the isosorbide has an amount ranging from 0.5 to 1.5 mol %.
8. The process as claimed in item 6, wherein the methyl 3-(3,5-di-tert-butyl-4-hydroxyphenyl)propionate has an amount ranging from 1.5 to 2.5 mol %.
9. The process as claimed in item 6, wherein the catalyst is selected from:
   (i) a tin based catalyst selected from the group consisting of stannous chloride (SC), dibutyl tin diacetate (DBTDA), dibutyl tin oxide (DBTO), dibutyl tin dialutarte (DBTDL), tin-2-ethylhexanoate (THE), butyl stannoic acid (BTA), butyltin hydroxide-oxide (BTA), and mixtures thereof;
   (ii) a salt of non-volatile inorganic acid selected from the group consisting of NaH₂PO₃, NaH₂PO₄, KH₂PO₄, CsH₂PO₄, Zn(CH₃CO₂)₂, and mixtures thereof; and
   (iii) sodium hydroxide, sodium carbonate, lithium hydroxide, lithium carbonate, tetraalkylammonium hydroxide, tetraalkyl ammonium carbonate, titanium alkoxide, lead alkoxide, metal acetate, 4-dimethyl amino pyridine, titanium alkoxide, synthetic hydrolacite, mixed metal oxide and mixtures thereof and has an amount ranging from 0.01 to 0.1 mol %.
10. The process as claimed in item 6, wherein the first solvent is selected from a group consisting of toluene, pentane, hexane, heptane, octane, cyclohexane, xylene and combination thereof and has an amount ranging from 15 to 25 mol %.
11. The process as claimed in item 6, wherein the step a) is carried out at a temperature ranging from 110 to 140 °C for a time period ranging from 10 to 14 hours and at a pressure ranging from 10 to 100 mbar.
12. The process as claimed in item 6, wherein the second solvent is selected from a group consisting of pentane, hexane, heptane, cyclo hexane, xylene, toluene and combination thereof and has an amount ranging from 35 to 45 mol %.
13. The process as claimed in item 6, wherein the second solvent is added to the crude product at a temperature ranging from 60 to 70 °C.
14. The process as claimed in item 6, wherein the adsorbent is selected from a group consisting of INF-04, silica gel, activated alumina, zeolites, synthetic polymeric resins and combination thereof and has an amount ranging from 2 to 3 mol %.
15. The process as claimed in item 6, wherein the step c) is carried out at a temperature ranging from 25 to 35 °C with stirring at a speed ranging from 50 to 200 RPM for a time period ranging from 30 to 90 minutes.
16. The process as claimed in item 6, wherein the adsorbent treatment of step c) is repeated until the acid impurity is reduced to less than 0.50 %.
17. The process as claimed in item 6, wherein the seeding agent is pure 3-(3,5-di-tert-butyl-4-hydroxy-phenyl)-propionic acid 6-[3-(3,5-di-tert-butyl-4-hydroxyphenyl)-propionyloxy]-hexahydro-furo[3,2-b]furan-3-yl ester and has an amount ranging from 0.001 to 0.005 mol%.
18. The process as claimed in item 6, wherein the stirring in step e) is carried out at a temperature ranging from 25 to 35 °C at a speed ranging from 50 to 200 RPM for a time period ranging from 6 to 24 hours.
19. The process as claimed in item 6, wherein the drying in step f) is carried out at a temperature ranging from 25 to 40 °C for a time period ranging from 12 to 24 hours at a pressure ranging from 10 to 100 mbar.
20. Use of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester for thermo-oxidative stabilization of polyolefins, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.
21. A composition for thermo-oxidative stabilization of polyolefins, said composition comprising:
   (a) a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73;
   (b) a secondary anti-oxidant; and
   (c) an acid scavenger.
22. The composition as claimed in item 21, wherein the composition comprises the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the secondary anti-oxidant in a weight ratio ranging from 10:1 to 1:10.
23. The composition as claimed in item 21, wherein the weight ratio between the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the acid scavenger ranges between 5.0:1.0 to 1.0:5.0.
24. The composition as claimed in item 21, wherein the secondary anti-oxidant is selected from phosphanite based secondary antioxidant and phosphite based secondary antioxidant.
25. The composition as claimed in item 21, wherein the acid scavenger is selected from hydrotalcite type acid scavengers, metal stearates type acid scavengers and mixtures thereof.

## Claims

1. A polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

2. The polymorphic Form B as claimed in claim 1, wherein the polymorphic Form B has a melting point ranging from 103 to 106 °C; or.
wherein the polymorphic Form B has a Themo gravimetric analysis (TGA) T_{d,5%} about 297 °C.

3. The polymorphic Form B as claimed in claim 1, wherein the polymorphic Form B has more than 85 % crystallinity, or.
wherein the polymorphic Form B is highly hydrophobic and having low moisture capability even after 12 to 18 months preservation.

4. A process of preparation of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, the process comprising:
a) heating an isosorbide and a methyl 3-(3,5-di-tert-butyl-4-hydroxyphenyl)propionate in presence of a catalyst and a first solvent to obtain a crude product;
b) adding a second solvent to the crude product to obtain a solution;
c) introducing an adsorbent to the solution with stirring to obtain a mixture;
d) filtering the mixture to obtain a filtrate;
e) adding a seeding agent in the filtrate with stirring to obtain a solid product; and
f) filtering the solid product followed by drying to obtain a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

5. The process as claimed in claim 4, wherein the isosorbide has an amount ranging from 0.5 to 1.5 mol %; or
wherein the methyl 3-(3,5-di-tert-butyl-4-hydroxyphenyl)propionate has an amount ranging from 1.5 to 2.5 mol %.

6. The process as claimed in claim 4, wherein the catalyst is selected from:
(i) a tin based catalyst selected from the group consisting of stannous chloride (SC), dibutyl tin diacetate (DBTDA), dibutyl tin oxide (DBTO), dibutyl tin dialutarte (DBTDL), tin-2-ethylhexanoate (THE), butyl stannoic acid (BTA), butyltin hydroxide-oxide (BTA), and mixtures thereof;
(ii) a salt of non-volatile inorganic acid selected from the group consisting of NaH₂PO₃, NaH₂PO₄, KH₂PO₄, CsH₂PO₄, Zn(CH₃CO₂)₂, and mixtures thereof; and
(iii) sodium hydroxide, sodium carbonate, lithium hydroxide, lithium carbonate, tetraalkylammonium hydroxide, tetraalkyl ammonium carbonate, titanium alkoxide, lead alkoxide, metal acetate, 4-dimethyl amino pyridine, titanium alkoxide, synthetic hydrolacite, mixed metal oxide and mixtures thereof and has an amount ranging from 0.01 to 0.1 mol %.

7. The process as claimed in claim 4, wherein the first solvent in step a) is selected from a group consisting of toluene, pentane, hexane, heptane, octane, cyclohexane, xylene and combination thereof and has an amount ranging from 15 to 25 mol %; or
wherein the step a) is carried out at a temperature ranging from 110 to 140 °C for a time period ranging from 10 to 14 hours and at a pressure ranging from 10 to 100 mbar.

8. The process as claimed in claim 4, wherein the second solvent in step b) is selected from a group consisting of pentane, hexane, heptane, cyclo hexane, xylene, toluene and combination thereof and has an amount ranging from 35 to 45 mol %; or
wherein the second solvent in step b) is added to the crude product at a temperature ranging from 60 to 70 °C.

9. The process as claimed in claim 4, wherein the adsorbent in step c) is selected from a group consisting of INF-04, silica gel, activated alumina, zeolites, synthetic polymeric resins and combination thereof and has an amount ranging from 2 to 3 mol %; or.
wherein the step c) is carried out at a temperature ranging from 25 to 35 °C with stirring at a speed ranging from 50 to 200 RPM for a time period ranging from 30 to 90 minutes; or.
wherein the adsorbent treatment of step c) is repeated until the acid impurity is reduced to less than 0.50 %.

10. The process as claimed in claim 4, wherein the seeding agent in step e) is pure 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and has an amount ranging from 0.001 to 0.005 mol%; or
wherein the stirring in step e) is carried out at a temperature ranging from 25 to 35 °C at a speed ranging from 50 to 200 RPM for a time period ranging from 6 to 24 hours.

11. The process as claimed in claim 6, wherein the drying in step f) is carried out at a temperature ranging from 25 to 40 °C for a time period ranging from 12 to 24 hours at a pressure ranging from 10 to 100 mbar.

12. Use of a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester for thermo-oxidative stabilization of polyolefins, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73.

13. A composition for thermo-oxidative stabilization of polyolefins, said composition comprising:
(a) a polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester, wherein the polymorphic Form B is crystalline, having an X-ray powder diffraction pattern (CuKα) comprising peaks at 2-theta about 8.48, 10.18, 12.28, 12.62, 13.42, 15.14, 16.39, 17.32, 17.76, 18.42, 19.12, 19.74, 20.89, 21.52, 23.58 and 24.73;
(b) a secondary anti-oxidant; and
(c) an acid scavenger.

14. The composition as claimed in claim 13, wherein the composition comprises the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the secondary anti-oxidant in a weight ratio ranging from 10:1 to 1:10; or
wherein the weight ratio between the polymorphic Form B of 3-(3,5-di-tert-butyl-4-hydroxy-phenyl)-propionic acid 6-[3-(3,5-di-tert-butyl-4-hydroxy-phenyl)-propionyloxy]-hexahydro-furo[3,2-b]furan-3-yl ester and the acid scavenger ranges between 5.0:1.0 to 1.0:5.0.

15. The composition as claimed in claim 13, wherein the secondary anti-oxidant is selected from phosphanite based secondary antioxidant and phosphite based secondary antioxidant; or
wherein the acid scavenger is selected from hydrotalcite type acid scavengers, metal stearates type acid scavengers and mixtures thereof.
